(19) Europäisches Patentamt
European Patent Office
Office européen des brevets

(11) Publication number: **0 104 908**
**B1**

(12) **EUROPEAN PATENT SPECIFICATION**

(45) Date of publication of patent specification: **27.12.90**

(21) Application number: **83305694.8**

(22) Date of filing: **23.09.83**

(51) Int. Cl.⁵: **C 07 C 43/29**, C 07 C 43/225, C 07 C 215/00, C 07 C 317/00, C 07 C 321/00, C 07 C 49/577, A 01 N 31/14, A 01 N 33/10, A 01 N 35/02, A 01 N 41/10

(54) **Arthropodicidal compounds.**

(30) Priority: **24.09.82 AU 6041/82**

(43) Date of publication of application:
**04.04.84 Bulletin 84/14**

(45) Publication of the grant of the patent:
**27.12.90 Bulletin 90/52**

(84) Designated Contracting States:
**BE CH DE FR GB IT LI NL SE**

(56) References cited:
**EP-A-0 094 085**
**AU-B- 502 950**
**FR-A-2 342 953**
**FR-A-2 481 695**
**US-A-4 206 230**
**US-A-4 218 468**

(73) Proprietor: **THE COMMONWEALTH SCIENTIFIC AND INDUSTRIAL RESEARCH ORGANISATION**
**Limestone Avenue**
**Campbell, Australian Capital Territory 2601 (AU)**

(72) Inventor: **Holan, George**
**86 Were Street**
**Brighton Victoria (AU)**
Inventor: **Johnson, Wynona Marguerite Phillips**
**3/102 Brewer Road**
**Bentleigh Victoria (AU)**
Inventor: **Rihs, Kurt**
**33 Stanhope Street**
**West Footscray Victoria (AU)**
Inventor: **Walser, Reimund August**
**11 Mitchell Road**
**Box Hill Victoria (AU)**

(74) Representative: **Lawrence, Peter Robin Broughton et al**
**GILL JENNINGS & EVERY 53-64 Chancery Lane**
**London WC2A 1HN (GB)**

**The file contains technical information submitted after the application was filed and not included in this specification**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to new compounds having arthropodicidal activity, to methods of preparing these compounds and to the use of the compounds as arthropodicides, especially as insecticides and acaricides.

The compounds provided by this invention have the general formula (I) or an isomeric form thereof:

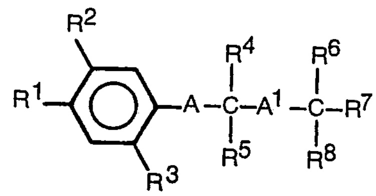

(I)

wherein

$R^1$ is a halo group; or a lower alkyl, lower alkoxy or lower alkylthio group, in each of which the alkyl group may be substituted with one or more halo groups;

$R^2$ is hydrogen or a halo or halomethyl group; or $R^1$ and $R^2$ together form a methylenedioxy, or a difluoro-methylenedioxy group or, $R^1$ and $R^2$ together with the carbon atoms to which they are attached, form an aromatic ring;

$R^3$ is hydrogen or a halo group;

A is one of the groups X or Y

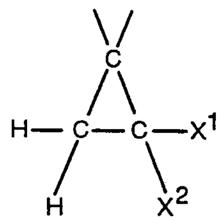

(X)

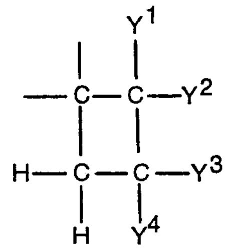

(Y)

wherein $X^1$ and $X^2$ are the same or different and each is a fluoro, chloro, bromo or methyl group, and $Y^1$, $Y^2$, $Y^3$ and $Y^4$ are the same or different and each is hydrogen or a fluoro, bromo, chloro, or methyl group;

$A^1$ is $CH_2$, $CF_2$, $CCl_2$, O, S, SO, NH or NZ

where Z is a halo group or

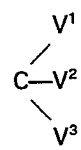

where $V^1$, $V^2$, $V^3$ are the same or different and each is fluorine, chlorine or hydrogen;

$R^4$ is hydrogen, chlorine or fluorine; and

$R^5$ is hydrogen, chlorine or fluorine; or

$R^4$ and $R^5$ together form =O when $A^1$ is $CH_2$; and

$R^6$ is hydrogen, deuterium, CN, or C≡CH; and

$R^7$ is 3-phenoxyphenyl, 2-phenoxy-6-pyridyl, 2-phenoxy-3-fluoro-6-pyridyl, pentafluorophenyl, 4-fluoro-3-phenoxyphenyl, N-pyrollyl-3-benzyl, 3,4-methylene-dioxy phenyl; or 3-(4-methoxyphenoxy)-phenyl;

$R^8$ is hydrogen or deuterium.

As used herein "halo" means fluoro, chloro or bromo; "lower" implies alkyl groups having from 1 to 4 carbon atoms. Alkyl groups having more than 2 carbon atoms may be straight or branched.

Preferred compounds are those in which $A^1$ is O, S, SO, NH or NZ and those in which $A^1$ is $CH_2$ and $R^4$ and $R^5$ form =O.

Related prior art compounds disclosed in our Australian Patent No. 502,950 and Australian Patent Application Nos. 42723/78 and 53362/79, are esters of acids of the general formula (II)

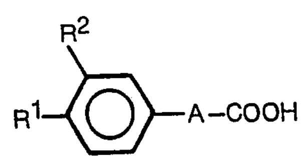

(II)

wherein A, $R^1$ and $R^2$ are essentially as defined above with one of the following alcohols:

3-phenoxybenzyl alcohol
2-benzyl-4-furylmethanol
α-cyano-3-phenoxybenzyl alcohol
3,4-methylenedioxybenzyl alcohol
α-ethynyl-3-phenoxybenzyl alcohol
α-cyano-3-(4-chlorophenoxy)benzyl alcohol

Bull and Searle in British patent 1,570,982 disclose *inter alia* insecticidal compounds of the structure (using the notation of formula (I))

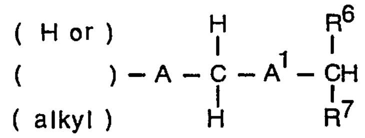

(III)

which are similar to the compound of this invention where $R^4 = R^5 = H$, $R^6 = H$, CN or C = CH, $R^7$ = 3-phenoxyphenyl or 3-(4-methoxyphenoxy)phenyl, $A^1$ = O or S and A is optionally substituted cycloalkyl. The major difference between these compounds is that they have a hydrogen or alkyl group attached to the group A instead of the substituted phenyl group

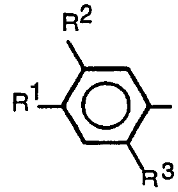

The same British Patent also discloses compounds of the structure:

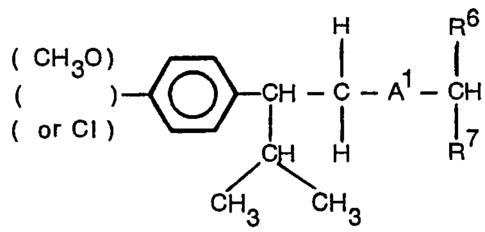

(IV)

which differ from the present compounds in that the cyclic group A is replaced by an isopropylmethylene group.

Bull and Searle in U.S. patent 4,073,812 claim very similar compounds in which the A group is a methylene group substituted with a branched chain alkyl of 3 to 6 carbon atoms and $R^7$ is 3-phenoxyphenyl or 2,3-(fluorine substited phenoxy)phenyl.

Japanese Patent application 56-154427 by Mitsui Toatsu Kagaku K.K. covers compounds of formula (V)

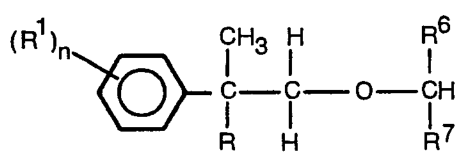

(V)

where $R^6$ = H and $R^7$ is 3-phenoxybenzyl, R is methyl or ethyl, and $R^1$ is halogen or lower alkyl.

These compounds are similar to the previously discussed compounds of Bull and Searle. In this case the cyclic group A has been replaced by $C(CH_3)_2$ or $C(CH_3)(C_2H_5)$ to produce quite different compounds to those of the present application.

U.S. Patent 4,218,468 to Mobil discloses ketone compounds where the A group of the present compounds is replaced by an isopropylmethylene group, $R^4$, $R^5$ together form a carbonyl oxygen and $A^1$ is $CH_2$, i.e., the compounds have the structure (VI).

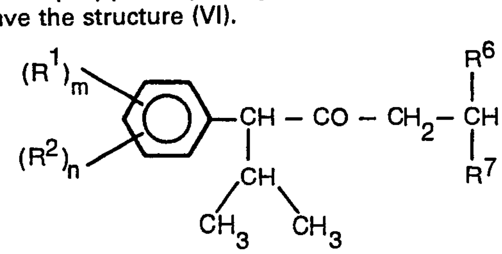

(VI)

It will be readily appreciated by those skilled in the art that the obvious structural differences between the prior art compounds (formulae (II) to (VI) and those of the present invention (formula (I)) will give rise to significant differences in insecticidal properties, both in level of activity in individual insect species and in the spectrum of activity against various species. Such differences, however, are not readily predictable either as to magnitude or sign. EP—A—0094085 (published after the priority date of the present invention and designating the Contracting States CH DE FR GB IT LI NL) discloses ether compounds of the formula X

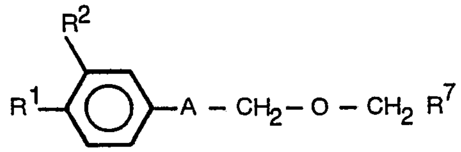

X

in which A may represent cyclopropyl or 2,2-difluoro cyclopropyl and in particular names 3'-phenoxybenzyl 1-(4-ethoxyphenyl)-2,2-difluorocyclopropyl-1-methyl ether; the disclosure of compounds in which A represents a 2,2-difluorocyclopropane derivative is entitled to an earliest priority date of 10th May 1983. Compounds of the present invention which are 2,2-di-substituted cyclopropane derivatives are entitled to the priority date of 24th September 1982. One compound disclosed in the citation is 3'-phenoxybenzyl-1-(4-ethoxyphenyl)-2,2-difluorocyclopropyl-1-methyl ether; the method to its preparation described therein inevitably led to the racemate 3'-phenoxybenzyl-1-(4-ethoxyphenyl)-2,2-difluorocyclopropyl-1-methyl ether in the present invention is mentioned for the first time on 23.09.83.

Prior national applications DE 3317908, NL 8301656, FR 2527203 and SE 8302464 include the specific disclosure of a process for producing the compound 1-(3-phenoxyphenyl)-3-[1-(4-chlorophenyl)-2,2-dichlorocyclopropyl]propane. There is no disclosure in the priority document of a process for separating the isomers of any of the compounds. The claims of the present application include a proviso disclaiming the racemate of that compound in so far as they cover those states but not the other four designated states.

The compounds of the invention (formula (I)) are optically active and can be resolved into their optical isomers by conventional methods. The invention thus includes the individual optical isomers of the compounds as well as the racemic forms.

The compounds of formula (I) may be prepared by the conventional methods of synthetic organic chemistry.

In general, the compounds are formed by reacting together compounds of the formulae (VIII) and (IX)

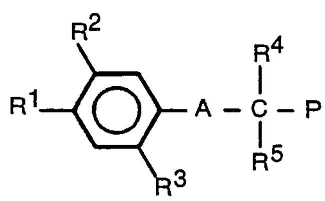

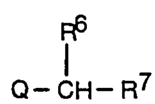

(VIII) (IX)

$R^1$, $R^2$, $R^3$, $R^4$, $R^5$, $R^6$ and A are as defined above and wherein P and Q are reactive atoms or groups, often referred to as "leaving groups", selected so that on reaction they give rise to the desired group $A^1$ or to another group which is convertible to that desired group.

By way of illustration, the compounds (I) having the $A^1$ groups listed below may be formed from the compounds (VIII) and (IX) having the indicated substituents P and Q. The groups indicated for P and Q could equally well be interchanged i.e. P = Br, Q = OH to give A' = —O—.

| $A^1$ | | P | Q |
|---|---|---|---|
| -O- | | -OH | Br- |
| -S- | (1) | -SH | Br- |
| -NH- | (2) | $-NH_2$ | Br- |
| -NZ- | | | |
| -CO- | | | |
| $-CH_2$ | (3) | -CHO | MgBr- |
| $-CCl_2$ | | | |
| $-CF_2$ | | | |

(1) Compounds wherein $A^1$ is —SO— can be prepared by oxidation of the corresponding thioether ($A^1$ = —S—).

(2) Compounds wherein $A^1$ is NZ are produced by reacting the amine with the halogen ZHal, where Hal is Cl, Br or I.

(3) The ketone ($A^1$ = —CO—) is a valuable intermediate for production of the compounds of the invention. Compounds where $A^1$ = —$CH_2$— are produced by reduction of the ketone, e.g., with dissolving metal. Compounds where $A^1$ = —$CCL_2$— or —$CF_2$— are produced by reacting the ketone with the appropriate chlorinating or fluroinating reagents, e.g., phosphorus pentachloride or diethylamine sulphur trifluoride, respectively.

The general method of the invention as stated above is illustrated by the following embodiments, which are representative of the methods which may be used for preparation of the compounds of the invention. It will be appreciated that the solvents, reagents, reaction conditions, work-up procedures and other reaction parameters specifically mentioned may be replaced by their known equivalents.

### General Method A

Preparation of ethers and sulphides ($A^1$ = —O— or —S—)

To a solution of sodium hydride (1 mmol) in anhydrous dimethoxyethane (DME) (1 ml) was added dropwise a solution of 1 mmol of the required 1-aryl-cycloalkyl-1-methyl carbinol or mercaptan (Formula VIII; P = —OH or —SH) in anhydrous DME (1 ml). This reaction mixture was stirred at room temperature for 0.75 hours. At the end of this time, a solution of 1 mmol of the required bromide (Formula IX; Q = Br) in anhydrous DME (0.5 ml) was added. The reaction was stirred at room temperature until thin layer chromatography indicated no starting material remained. The reaction mixture was then filtered, the filtrate concentrated and the residue chromatographed on silica gel using petroleum spirit (40—60°)/ethyl acetate mixtures as the eluting solvents.

Examples of compounds prepared by this method were:

3′-phenoxybenzyl 1-(4-ethoxyphenyl)-2,2-difluorocyclopropyl-1-methyl ether.

3′-Phenoxbenzyl 1-(4-ethoxyphenyl)-2,2-difluorocyclopropyl-1-methyl sulphide.

### General Method B

Preparation of α-cyano sulphides ($A^1$ = —S—, $R^6$ = —CN)

In an atmosphere of argon the 1-aryl-1-cycloalkylmethyl mercaptan (Formula VIII; P = —SH) (1.0 mmol) was diluted with acetone (1 ml). To this solution with stirring, triethylamine (0.1 g) was added followed by α-bromo-3-phenoxy benzonitrile (1 mmol) which was added dropwise with stirring. After the initial exothermic reaction subsided the reaction mixture was stirred for an additional 16 hours. It was then diluted with diethyl ether (10 ml) and the precipitate of amine hydrobromide filtered off and washed with additional ether.

The ether filtrates were evaporated and the residual oil purified by HPLC using 5:95 ethyl acetate/pet. ether (60°—40°) as the eluent.

Example: α-cyano-3′-phenoxybenzyl 1-(4-ethoxyphenyl)-2,2-difluorocyclopropyl-1-methyl sulphide.

### General Method C

Preparation of Sulphoxides (A′ = —SO—)

The sulphoxide was formed by oxidation of the sulphide (prepared according to Method A) by conventional methods using either 3-chlorobenzoic acid or hydrogen peroxide-acetic acid oxidants.

Example: 3′-phenoxybenzyl 1-(4-ethoxyphenyl)-2,2-difluorocyclopropyl-1-methyl-sulphoxide. Melting point 105.4°C.

### General Method D

Preparation of aralkyl 1-aryl-halocycloalkyl-1-carboxamides (A′ = —NH; $CR^4R^5$ = —CO—)

To a 2-necked round bottom flask under $N_2$ was added a 20% (w/w) suspension of potassium hydride (2.2 mmol) in oil. The oil was removed by washing 3 times with petroleum spirit (30—40°). The residual petroleum spirit was removed in vacuo and under dry $N_2$ the powdered potassium hydride was resuspended in dry tetrahydrofuran (5 ml) and cooled to 0°. At 0.5°C the 1-aryl-halocycloalkyl-1-carboxamide (Formula VIII; $CR^4R^5P$ = $CONH_2$) (2 mmol) was added in small portions over 10 minutes. To the now clear solution 18-crown-6 (3 mg) was added, followed by the solution of the aralkylhallide (Formula IX; Q = Hal) (2.2 mmol) in dry tetrahydrofuran (2 ml). The reaction was stirred at room temperature for 1 hour and under reflux for a further 16 hours. After cooling to room temperature the resulting suspension was filtered, washed with dry ether (10 ml) and the combined filtrates evaporated to dryness. The crude product was chromatographed over a short silica gel column using dichloromethane as the eluent.

Example: 3′-phenoxybenzyl 1-(4-ethoxy phenyl)-2,2-difluorocyclopropyl-1-carboxamide. The resulting product was recrystallized from dichloromethane-petroleum spirit (40—60°C) and recovered as a white solid in 67.4% yield, m.p. 89.1°C.

General Method E

Preparation of aralkyl 1-aryl-halocycloalkylmethyl amines ($A^1$ = —NH—; $CR^4R^5$ = —$CH_2$—)

To a cold (0°C), stirred solution of diborane (2 mmol) in dry tetrahydrofuran (2 ml), under dry nitrogen, aralkyl-1-aryl-halocycloalkyl-1-carboxamide (0.5 mmol) was added. (Formula I; $CR^4R^5$ P = —$CONH_2$). The mixture was heated and stirred under reflux for 1 hour and at room temperature for an additional 16 hours. To the reaction mixture aqueous hydrochloric acid (2 ml) was added and most of the tetrahydrofuran removed by distillation. The residue was quenched with ice (50 g) and made basic with sodium hydroxide. After extraction with diethylether the combined ether extracts were washed with distilled water and NaCl solution. After separation the solvent layer was dried over anhydrous $Na_2SO_4$. Filtration and removal of the solvent in vacuo gave the product as an oil in >90% yield.

Example: 3'-phenoxybenzyl 1-(4-ethoxyphenyl)-2,2-difluorocyclopropylmethylamine:

A sample was purified by prep. HPLC on a silica gel column using 5% ethylacetate in dichloromethane as the eluent. A faint yellow oil was recovered in 57% yield.

General Method F

Preparation of α-cyanoamines ($A^1$ = —NH—, $R^6$ = —CN)

Under cover of nitrogen, 3-phenoxybenzaldehyde (238 mg, 1.2 mmol) was added under stirring to the appropriate amine (Formula VIII; $A^1$ = $NH_2$) (0.95 mmol) dry potassium cyanide (77 mg, 1.2 mmol), glacial acetic acid (0.069 ml, 1.2 mmol) and dry methanol (3 mls). The reaction mixture was stirred for 48 hours at room temperature.

After this period of time, the methanol was recovered in vacuo and sodium hydroxide (10% solution) was added until the reaction mixture was strongly alkaline. The aqueous mixture was extracted (4×) with dichloromethane (5 mls). The dichloromethane extracts were combined, washed with water and brine and dried over anhydrous sodium sulfate. The solvent was removed in vacuo.

The product was passed through a short silica gel column to remove some of the remaining aldehyde.

The product was then purified by preparative high performance liquid chromatography on silica gel using 15% ethylacetate/petroleum ether (b.p. 40—60°C).

Example: α-cyano-3'-phenoxybenzyl 1-(4-ethoxyphenyl)-2,2-difluorocyclopropyl-1-methylamine.

General Method G

Preparation of tertiary amines ($A^1$ = —NZ—)

Under nitrogen atmosphere formic acid (0.15 ml, 4.0 mmol) was added to the secondary amine (0.735 mmol) (e.g. Formula VIII; P = —$NHCH_3$). Formaldehyde (0.14 ml, 5.0 mmol) was then added to this reaction mixture. This was treated to 90°C for 5 hours and then at room temperature for 14 hours.

Sodium hydroxide (10% solution) was then added until the reaction mixture was alkaline. The aqueous mixture was extracted with diethyl ether (6 × 6 ml). The diethyl ether extracts were combined, washed with water and sodium chloride solution and dried over sodium sulfate. The solvent was removed in vacuo.

The resultant oil was then purified by preparative high performance liquid chromatography on silica gel column using ethyl acetate/petroleum ether (b.p. 40—60°C) (20:80) to obtain pure product amine.

Example: 3'-phenoxybenzyl 1-(4-ethoxyphenyl)-2,2-difluorocyclopropyl dimethylamine.

General Method H

Preparation of Aryl-1-Arylcycloalkyl-1-propan-1-ones ($A^1$ = —$CH_2$—; $R^4$+$R^5$ = =O)

To aryl-ethylbromomagnesium bromide (Formula IX; Q = MgBr) (5 mmol) in ether (10 ml), cadmium chloride (2.75 mmol) was added over 5 minutes at 5°C. The reaction mixture was stirred for 1 hour while warming to room temperature. The ether was replaced by distillation with dry benzene and cooled in ice. To this solution the corresponding 1-aryl-1-cycloalkyl acid chloride (Formula VIII; $CR^4R^5$P = —COCl) (5 mmol) dissolved in dry benzene (10 ml) was added at once. The reaction mixture was allowed to warm to room temperature over 1 hour. The reaction mixture was then quenched in dilute HCl and the organic layer washed subsequently with water, $NaHCO_2$ solution and again with water. After drying with anhydrous $Na_2SO_4$ the solvent was evaporated. The residual oil was chromatographed on an HPLC silica gel column using pet. ether (60°—80°)/$CH_2Cl_2$/diethyl ether (33.3:33.3:33.3) as eluents to give the 1-propanone.

Example: 3-(3-phenoxyphenyl-1-[1-(4-ethoxyphenyl)-2,2,3,3-tetrafluorocyclobutyl]-propan-1-one.

The active compounds of the invention are well tolerated by plants, have a favourable level of toxicity to warm-blooded animals, and can be used for combating arthropod pests, especially insects or acarids, which are encountered in agriculture, in veterinary practice, in forestry, in the protection of stored products and of materials, and in the hygiene field. They are active against normally sensitive and resistant species and against all or some stages of development. The abovementioned pests include *Lucilia cuprina* (the Australian sheep blowfly), *Blatella germanica* (German cockroach), *Periplaneta americana* (American cockroach) and *Heliothis punctigera* (cotton budworm).

The present invention also provides arthropodicial compositions containing as active ingredients a compound of the present invention.

The present invention also provides a method of combating arthropods (especially insects or acarids) which comprises applying to the arthropods, or to a habitat thereof, a compound of the present invention alone or in the form of a composition containing as active ingredient a compound of the present invention.

The present invention also provides a method of freeing or protecting domesticated animals from parasitical insects or acarids which comprises applying to said animals a compound according to the present invention, or a composition containing such a compound as the active ingredient.

In the compositions of this invention, the active compounds are converted into such customary formulations as solutions, emulsions, wettable powders, suspensions, powders, dusting agents, foams, pastes, soluble powders, granules, aerosols, suspension-emulsion concentrates, seed-treatment powders, natural and synthetic materials impregnated with active compound, very fine capsules in polymeric substances, and coating compositions for use on seed, as well as ULV cold mist and warm mist formulations.

These formulations may be produced in known manner, for example by mixing the active compounds with extenders, i.e., liquid or liquefied gaseous or solid diluents or carriers, optionally with the use of surface-active agents, i.e., emulsifying agents and/or dispersing agents and/or foam-forming agents. Where water is used as an extender, auxiliary solvents, such as for example, organic solvents, can also be used.

Examples of suitable liquid diluents or carriers, especially solvents, are aromatic hydrocarbons, such as xylene, toluene or alkyl naphthalenes, chlorinated aromatic or chlorinated aliphatic hydrocarbons, such as chlorobenzenes, chloroethylenes or methylene chloride; aliphatic or alicyclic hydrocarbons, such as cyclohexane or paraffins, for example mineral oil fractions; alcohols, such as butanol or glycol, as well as their ethers; and esters, ketones, such as acetone, methyl ethyl ketone, methyl isobutyl ketone or cyclohexanone; and strongly polar solvents, such as dimethylformamide and dimethyl sulphoxide, as well as water.

By liquefied gaseous diluents or carriers are meant liquids which would be gaseous at normal temperature and under normal pressure, for example aerosol propellants, such as halogenated hydrocarbons as well as butane, propane, nitrogen and carbon dioxide.

Examples of solid carriers are ground natural minerals, such as kaolins, clays, talc, chalk, quartz, attapulgite, montmorillonite or diatomaceous earth; and ground synthetic minerals, such as highly-dispersed silicic acid, alumina and silicates. As solid carriers for granules there may be used crushed and fractionated natural rocks such as calcite, marble, pumice, sepiolite and dolomite; as well as synthetic granules of inorganic and organic meals, and granules of organic material such as sawdust, coconut shells, maize cobs and tobacco stalks.

Examples of emulsifying and/or foam-forming agents are non-ionic and anionic emulsifiers, such as polyoxyethylene-fatty acid esters, polyoxy ethylene-fatty alcohol ethers, for example alkylaryl polyglycol ethers, alkyl sulphonates, alkyl sulphates, aryl sulphonates, as well as albumin hydrolysis products. Dispersing agents include, for example, lignin sulphite waste liquors and methylcellulose.

Adhesives such as carboxymethylcellulose and natural and synthetic polymers in the form of powders, granules or latices, such as gum arabic, polyvinyl alcohol and polyvinyl acetate, can be used in the formulations.

It is also possible to use colorants such as inorganic pigments, for example iron oxide, titanium oxide and Prussian Blue, and organic dyestuffs, such as alizarin dyestuffs, azo dyestuffs or metal phthalocyanine dyestuffs.

The formulations in general will contain from 0.1 to 95 per cent by weight of active compound, preferably from 0.5 to 90 per cent by weight.

The active compounds according to the invention may be used in the form of formulations of the types that are commercially available or in the use forms prepared from these formulations.

The active compound content of the use forms prepared from the formulations of the types that are commercially available can vary within wide ranges. The active compound concentration of the use forms can be from 0.0000001 to 100% by weight of active compound, preferably from 0.0001 to 10% by weight.

The compounds may be employed in a customary manner appropriate for the particular use forms.

The active compounds according to the invention are also suitable for combating ectoparasites and endoparasites in the field of veterinary medicine.

The compounds may be used in a known manner, such as orally in the form of, for example, tablets, capsules, drenches and granules; dermally by means of, for example, dipping, spraying, pouring-on, spotting-on and powdering.

The compounds may be employed either as the sole toxic agent in compositions such as those described above, or in combination with other insecticides such as pyrethrum, rotenone, or with fungicidal or bactericidal agents, to provide compositions useful for household and agricultural dusts and sprays, textile coating and impregnation, and the like.

In particular, the compounds of the invention may be advantageously combined with other substances which have a synergistic or potentiating action. Generally such substances are of the class of microsomal oxidase inhibitors, i.e., they inhibit the detoxification of insecticides in insects produced by the action of oxidative enzymes. Typical substances of this type are the pyrethrin synergists of which the following are examples:

[2-(2-butoxyethoxy)ethoxy]-4,5-methylenedioxy-2-propyltoluene (Piperonyl butoxide), 3-hexyl-5(3,4-methylenedioxyphenyl)-2-cyclohexanone (Piperonyl cyclonene), 2-(3,4-methylenedioxyphenoxy)-3,6,9-trioxaundecane (Sesoxane or Sesamex), 1,2-(methylenedioxy)-4-[2-(octylsulfinyl)propyl]benzene

(Sulfoxide), dipropyl-5,6,7,8-tetrahydro-7-methylnaphtho-[2,3-d], 3-dioxole-5,6-dicarboxylate (n-Propyl isome), as well as propynyl ethers and propynyl oximes.

("Sesoxane", "Sesamex" and "Sulphoxide" are Registered Trade Marks).

Piperonyl butoxide is particularly useful as a potentiator. The amount of piperonyl butoxide used may vary from 1/100th to fifty times the weight of the compound I the preferred range being from about 1/100th to five parts by weight. 'Sesamex' also is a useful potentiator in similar amounts.

The preparation and properties of the compounds of the invention are illustrated by the following specific examples. It should be noted, of course, that these examples are intended to be illustrative of the methods and procedures utilized in preparing the compounds and that they are not intended to be restrictive or to be regarded as embodying the only way in which the compounds can be formed and recovered.

Examples of formulations in accordance with the invention are as follows: (parts are by weight):

*Dusts*

The following substances are used to produce (a) a 5% dust and (b) a 2% dust:

(a) 5 parts of active substance,
95 parts of talcum;

(b) 2 parts of active substance,
1 part of highly dispersed silicic acid,
97 parts of talcum.

The active substance is mixed and ground with the carriers.

*Granulates*

The following ingredients are used to produce a 5% granulate:

5 parts of active substance,
0.25 part of epichlorohydrin,
0.25 parts of cetyl polyglycol ether,
3.50 parts of polyethylene glycol,
91 parts of kaolin.

The active substance is mixed with epichlorohydrin and dissolved with 6 parts of acetone, the polyethylene glycol and cetyl polyglycol ether are then added. The solution obtained is sprayed onto kaolin and the acetone is evaporated off in vacuo.

*Wettable powders*

The following constituents are used to produce (a) a 40%, (b) and (c) a 25%, and (d) a 10% wettable powder:

(a) 40 parts of active substance,
5 parts of sodium lignin sulphonate,
1 part of sodium dibutyl-naphthalene sulphonate,
54 parts of silicic acid;

(b) 25 parts of active substance,
4.5 parts of calcium lignin sulphonate,
1.9 parts of chalk/hydroxyethyl cellulose mixture (1:1)
1.5 parts of sodium dibutyl-naphthalene sulphonate,
19.5 parts of silicic acid,
19.5 parts of chalk,
28.1 parts of kaolin;

(c) 25 parts of active substance,
2.5 parts of isooctylphenoxy-polyoxyethylene ethanol,
1.7 parts of chalk/hydroxyethyl cellulose mixture (1:1),
8.3 parts of sodium aluminium silicate,
16.5 parts of keiselguhr,
46 parts of kaolin;

(d) 10 parts of active substance,
3 parts of a mixture of the sodium salts of saturated fatty alcohol sulphates,
5 parts of naphthalenesulphonic acid/formaldehyde condensate,
82 parts of kaolin.

The active substance is intimately mixed in suitable mixers with the additives, and the mixture is then ground in the appropriate mills and rollers to obtain wettable powders which can be diluted with water to give suspensions of the desired concentration.

*Emulsifiable Concentrates*

The following substances are used to produce (a) a 10%, (b) a 25%, and (c) a 50% emulsifiable concentrate:

(a) 10 parts of active substance,
3.4 parts of epoxidised vegetable oil,
3.4 parts of a combination emulsifier consisting of fatty alcohol polyglycol ether and alkylarylsulphonate calcium salt,
40 parts of dimethylformamide,
43.2 parts of xylene;

(b) 25 parts of active substance,
2.5 parts of epoxidised vegetable oil,
10 parts of alkylarylsulphonate/fatty alcohol polyglycol ether mixture,
5 parts of dimethylformamide,
57.5 parts of xylene;

(c) 50 parts of active substance,
4.2 parts of tributylphenol-polyglycol ether,
5.8 parts of calcium-dodecylbenzenesulphonate,
20 parts of cyclohexanone,
20 parts of xylene.

Emulsions of the required concentration can be prepared from these concentrates by dilution with water.

*Sprays*

The following constituents are used to produce (a) a 5% spray, (b) a 95% spray, and (c) a synergised 4% spray.

(a) 5 parts of active substance,
1 part of epichlorohydrin,
94 parts of ligroin (boiling limits 160°—190°C);

(b) 95 parts of active substance,
5 parts of epichlorohydrin;

(c) 4 parts of active substance,
1 part of piperonyl butoxide,
79 parts of deodorised kerosene,
16 parts of alkylated naphthalene.

The invention is further illustrated by the following Examples

Examples 1—18

Preparation of Compounds

The compounds shown in Table 1 were prepared using the appropriate General Method (A through H) as described above. $R^3$ is hydrogen in all the compounds.

Table 1A gives analytical data for the compounds. In Table 1B, the nmr resonances are identified as follows:

(i) Hydrogen atoms at $C_3$ of cyclopropyl ring (X) or $C_4$ of cyclobutyl ring (Y).
(ii) $R^4$, $R^5$ = H.
(iii) $R^6$ = H.

TABLE 1

| Example No. | | $R^1$ | $R^2$ | A* | $R^4$ | $R^5$ | $A^1$ | $R^6$ | $R^{7**}$ | $R^8$ |
|---|---|---|---|---|---|---|---|---|---|---|
| | 1 | $C_2H_5O$ | H | $Cl_2$ | H | H | O | H | PB | H |
| | 2 | $C_2H_5O$ | H | TF | H | H | O | H | PB | H |
| | 3 | $C_2H_5O$ | H | $F_2$ | H | H | O | H | PB | H |
| (+)isomer of 3 | 4 | $C_2H_5O$ | H | $F_2$ | H | H | O | H | PB | H |
| (−)isomer of 3 | 5 | $C_2H_5O$ | H | $D_2$ | H | H | O | H | PB | H |
| | 6 | $C_2H_5O$ | H | $F_2$ | H | H | O | H | PBF | H |
| | 7 | $C_2H_5O$ | H | $F_1Cl$ | H | H | O | H | PB | H |
| | 8 | Cl | H | $F_2$ | H | H | O | H | PB | H |
| | 9 | Cl | Cl | TF | H | H | O | H | PB | H |
| | 10 | $C_2H_5O$ | H | $Cl_2$ | H | H | O | H | PF | H |
| | 11 | $C_2H_5O$ | H | $F_2$ | H | H | NH | H | PB | H |
| | 12 | $C_2H_5O$ | H | $F_2$ | H | H | $NCH_3$ | H | PB | H |
| | 13 | $C_2H_5O$ | H | $F_2$ | H | H | NH | CN | PB | H |
| | 14 | $C_2H_5O$ | H | $Cl_2$ | H | H | NH | H | PB | H |
| | 15 | $C_2H_5O$ | H | $F_2$ | H | H | S | H | PB | H |
| | 16 | $C_2H_5O$ | H | $F_2$ | H | H | S | CN | PB | H |
| | 17 | $C_2H_5O$ | H | $F_2$ | H | H | SO | CN | PB | H |
| | 18 | $C_2H_5O$ | H | TF | —O— | | $CH_2$ | H | PB | H |

* Key for A
$Cl_2$ = dichlorocyclopropane
$F_2$ = difluorocyclopropane
$F_1Cl$ = fluorochlorocyclopropane
TF = Tetrafluorocyclobutane
$R^7$ PB = 3 phenoxyphenyl
PBF = 4-fluoro-3-phenoxyphenyl
PF = pentafluorophenyl

TABLE 1A

| Example No. | | FOUND % | | | | | CALCULATED % | | | | |
|---|---|---|---|---|---|---|---|---|---|---|---|
| | | C | H | F | Cl | N(S) | C | H | F | Cl | N(S) |
| | 1 | 68.1 | 5.5 | — | 15.8 | — | 67.7 | 5.5 | — | 16.0 | — |
| | 2 | 68.6 | 5.2 | 15.4 | — | — | 67.8 | 5.3 | 16.5 | — | — |
| | 3 | 72.9 | 5.5 | 9.0 | — | — | 73.2 | 5.9 | 9.3 | — | — |
| (+)isomer of 3 | 4 | SAME AS NO. 3 MOL. ROTATION $[\alpha]_D^{14}$ = +13.073 (X = 1.0; $CHCl_3$) | | | | | | | | | |
| (−)isomer of 3 | 5 | SAME AS NO. 3 MOL. ROTATION $[\alpha]_D^{14}$ = −13.74 (C = 1.0; $CHCl_3$) | | | | | | | | | |
| | 6 | 70.1 | 5.6 | 13.0 | — | — | 70.1 | 5.4 | 13.3 | — | — |
| | 7 | 70.3 | 5.8 | 4.8 | 8.4 | — | 80.3 | 5.7 | 4.5 | 8.3 | — |
| | 8 | 69.3 | 4.8 | 9.7 | 8.8 | — | 68.9 | 4.8 | 9.5 | 8.8 | — |
| | 9 | 59.6 | 3.9 | 15.2 | 15 | — | 59.4 | 3.7 | 15.7 | 14.6 | — |
| | 10 | 51.8 | 3.5 | 21.7 | 16.2 | — | 51.7 | 3.4 | 21.5 | 16.0 | — |
| | 11 | 73.6 | 5.9 | 9.1 | — | 3.6 | 73.3 | 6.2 | 9.3 | — | 3.5 |
| | 12 | 73.7 | 6.3 | 9.2 | — | 3.5 | 73.7 | 6.4 | 9.0 | — | 3.3 |
| | 13 | 71.9 | 5.6 | 8.7 | — | 6.4 | 71.9 | 5.6 | 8.7 | — | 6.4 |
| | 14 | 67.5 | 5.8 | — | 15.8 | 3.2 | 67.9 | 5.7 | — | 16.0 | 3.2 |
| | 15 | 70.1 | 5.4 | 8.9 | — | (7.7) | 70.4 | 5.4 | 8.9 | — | (7.5) |
| | 16 | 69.3 | 4.9 | 8.6 | — | (7.1) | 69.2 | 5.1 | 8.4 | — | (7.1) |
| | 17 | 65.3 | 5.2 | 8.5 | — | (7.2) | 65.5 | 5.3 | 8.3 | — | (7.0) |
| | 18 | 68.3 | 5.3 | 16.0 | — | — | 68.6 | 5.1 | 16.0 | — | — |

TABLE 1B

| Example No. | | (i) | (ii) | (iii) |
|---|---|---|---|---|
| | 1 | 1.86, 1.71 (ABq) J=7.5 Hz | 3.96 (s) | 4.4 (s) |
| | 2 | 2.4—3.1 (2H) | 3.5 (2H) | 4.15 (2H) (s) |
| | 3 | 1.71—1.33 (m) | 3.65 (t) J=2 Hz | 4.39 (s) |
| (+)isomer of 3 | 4 | | SAME AS NO. 3 | |
| (−)isomer of 3 | 5 | | SAME AS NO. 3 | |
| | 6 | 1.72—1.32 (m) | 3.62 (t) J=2 Hz | 4.32 (s) |
| | 7 | 2.04—1.24 (m) | 3.69 (m) $W_{Y_2}$ = 8 Hz | 4.44 (s) |
| | 8 | 1.75—1.47 (m) | 3.63 s | 4.37 (s) |
| | 9 | 2.4—3.05 (2H) | 3.63 (2H) | 4.32 (2H) s |
| | 10 | 1.83, 1.72 (ABq) J=5.5 Hz | 3.82 (s) | 4.45 (t) J=2 Hz |
| | 11 | 1.13—1.68 (m) | 2.75 (t) J=2 Hz | 3.57 (s) |
| | 12 | 1.87—1.2 (m) | 2.68 (m) $W_{Y_2}$ =8 Hz | 3.4 (d) J=4 Hz |
| | 13 | 1.78—1.35 (m) | 3.0 (t) J=2 Hz | 4.63 (d) J=5 Hz |
| | 14 | 1.62—1.81 (ABq) J=7.5 Hz | 2.79—3.16 (ABq) J=12 Hz | 3.62 (s) |
| | 15 | 1.4—2.0 (2H) | 2.72 (2H) | 3.30 (2H) s |
| | 16 | 1.3—1.95 (2H) | 2.7—3.6 (2H) | 4.12 (1H) s |
| | 17 | 1.7—2.1 (2H) | 2.7—3.6 (2H) | (d) [4.33] (1H) |
| | 18 | | Infrared spectrum —C—O = 1735 $cm^{-1}$ | |

The arthropodicidal activities of the compounds were determined according to the following Examples 19—21.

Example 19

Insecticidal activity was investigated against blowfly, *Lucilia cuprina.* The method used was a follows:

(a) The compounds were tested for activity against a dieldrin susceptible strain (BLL) which had been collected before dieldrin usage in the field.

The test compound was applied in acetone solution, 0.5 µl dispensed with a Drummond micropipette to the dorsum of the thorax of 2—3 day old females. Adult flies were fed on water and sugar-only and maintained at 25°C and 60—70% RH. The mortalities were determined after 24 hours. Moribund flies were regarded as dead. The $LD_{59}$ values, in terms of concentration, were interpolated from the probit/log dose relation using a computer program.

The results given in Table 2 are expressed in terms of the *Potency Index* which is the ratio $LD_{50}$ Permethrin/$LC_{50}$ Test Compound.

(b) *Potentiation*

The compound was also tested on the insects described above in conjunction with the potentiator piperonyl butoxide by pretreating each insect with 1 µl of a 2% solution of the potentiator in acetone.

The mortalities were counted at 48 hours after treatment and compared with acetone and acetone/potentiator controls.

The $LD_{50}$ values were determined as described above and converted to Potency Index values.

About the same levels of potentiation were obtained when piperonyl butoxide was replaced by an equal amount of "Sesoxane".

The results are shown in Table 2.

Example 20

Insecticidal activity against the German cockroach (*Blatella germanica*) was determined using the following method:

The compound under test was applied in acetone solution at a range of concentrations. 0.5 µl was dispensed with a Drummond micropipette to the ventral thorax of adult cockroaches. The mortalities were determined after 48 hours. Moribund cockroaches were regarded as dead. The $LD_{50}$ values in terms of concentration were determined by probit analysis of the mortality/concentration data and converted to Potency Index values.

The results are shown in Table 2.

Example 21

Insecticidal activity against the cotton pest *Heliothis punctigera* was determined using the following method:

The compound under test was applied in acetone solution at a range of concentrations. 0.5 µl was dispensed with a Drummond micropipette to the dorsal surface of 3rd instar larvae. Each larva was held in a separate container and was fed on spinach and maintained at 25°C and 60—70% RH. The mortalities were determined after 48 hours. Moribund larvae were regarded as dead. The $LD_{50}$ values in terms of concentration were determined by a probit analysis of the mortality/concentration data and converted to Potency Index values.

The results are given in Table 2.

TABLE 2

POTENCY INDEX (Permethrin = 100)

| Example No. | | Lucilia cuprina | | Blatella germanica | Heliothis punctigera |
|---|---|---|---|---|---|
| | | cpd. alone | + Synergist | | |
| | 1 | 9 | 7 | | 42 |
| | 2 | 10 | 8 | 19 | 9 |
| | 3 | 58 | 46 | 280 | 394 |
| (+)isomer of 3 | 4 | 245 | 150 | — | — |
| (−)isomer of 3 | 5 | 44 | 67 | — | — |
| | 6 | 58 | 100 | | 591 |
| | 7 | 89 | 43 | 37 | 25 |
| | 8 | 246 | 60 | — | 0.1 |
| | 9 | 3 | 1 | — | — |
| | 10 | 3 | 7 | — | — |
| | 11 | 2 | 1 | — | 35 |
| | 12 | 0.3 | 1 | — | — |
| | 13 | 7 | 64 | — | — |
| | 14 | 0.43 | 1 | — | — |
| | 15 | 109 | 2000 | 11 | 650 |
| | 16 | 6 | 53 | — | — |
| | 17 | 0.3 | 3 | — | — |
| | 18 | 2 | 2 | 0.2 | — |

**Claims for the Contracting States: BE CH GB IT**

1. A compound of the general formula (I) or an isomeric form thereof:

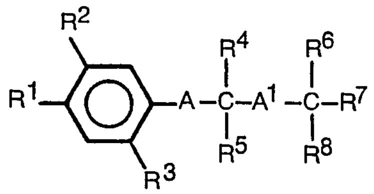

(I)

wherein

$R^1$ is a halo group; or a lower alkyl, lower alkoxy or lower alkylthio group, in each of which the alkyl group may be substituted with one or more halo groups;

$R^2$ is hydrogen or a halo or halomethyl group; or $R^1$ and $R^2$ together form a methylenedioxy, or a difluoro-methylenedioxy group or $R^1$ and $R^2$ together with the carbon atoms to which they are attached form an aromatic ring;

$R^3$ is hydrogen or a halo group;

A is one of the groups X or Y

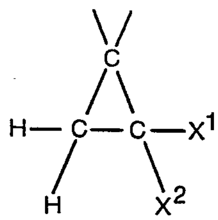

(X)

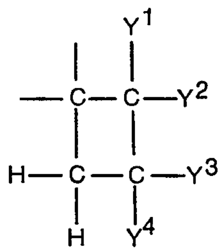

(Y)

wherein $X^1$ and $X^2$ are the same or different and each is a fluoro, chloro, bromo or methyl group, and $Y^1$, $Y^2$, $Y^3$ and $Y^4$ are the same or different and each is hydrogen or a fluoro, bromo, chloro, or methyl group;

$A^1$ is $CH_2$, $CF_2$, $CCl_2$, O, S, SO, NH or NZ where Z is a halo group or

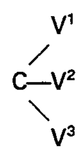

where $V^1$, $V^2$, $V^3$ are the same or different and each is fluorine, chlorine or hydrogen;

$R^4$ is hydrogen, chlorine or fluorine; and

$R^5$ is hydrogen, chlorine or fluorine; or

$R^4$ and $R^5$ together form =O when $A^1$ is $CH_2$; and

$R^6$ is hydrogen, deuterium, CN, or C≡; and

$R^7$ is 3-phenoxyphenyl, 2-phenoxy-6-pyridyl, 2-phenoxy-3-fluoro-6-pyridyl, pentafluorophenyl, 4-fluoro-3-phenoxyphenyl, N-pyrollyl-3-benzyl, 3,4-methylene-dioxy phenyl; or 3-(4-methoxyphenoxy)-phenyl;

$R^8$ is hydrogen or deuterium.

2. A compound as claimed in claim 1 characterized in that group $A^1$ is —O—.

3. A compound as claimed in claim 1 characterized in that the group $A^1$ is —S—.

4. A compound as claimed in claim 1 characterized in that the group $A^1$ is —SO—.

5. A compound as claimed in claim 1 characterized in that the group $A^1$ is —NH— or —NZ—.

6. A compound as claimed in claim 1 characterized in that the group $A^1$ is —$CH_2$— and $R^4$ and $R^5$ together form =O.

7. Compound according to claim 1 selected from:

3'-phenoxybenzyl-1-(4-ethoxyphenyl)-2,2,3,3-tetrafluorocyclobutyl-1-methyl ether;
3'-phenoxybenzyl-1-(4-ethoxyphenyl)-2,2-dichlorocyclopropyl-1-methyl ether;
4'-fluoro-3'-phenoxybenzyl-1-(4-ethoxyphenyl)-2,2-difluoro-cyclopropyl-1-methyl ether;
3'-phenoxybenzyl-1-(4-ethoxyphenyl)-2-fluoro-2-chlorocyclopropyl-1-methyl ether;
3'-phenoxybenzyl-1-(4-chlorophenyl)-2,2-difluorocyclopropyl-1-methyl ether;
3'-phenoxybenzyl-1-(3,4-dichlorophenyl)-2,2,3,3-tetrafluorocyclobutyl-1-methyl ether;
pentafluorophenylmethyl-1-(4-ethoxyphenyl)-2,2-dichlorocyclopropyl-1-methyl ether;
3'-phenoxybenzyl-1-(4-ethoxyphenyl)-2,2-difluorocyclopropyl-1-methyl amine;

N-methyl-3'-phenoxybenzyl-1-(4-ethoxyphenyl)-2,2-difluorocyclopropyl-1-methyl amine;
α-cyano-3'-phenoxybenzyl-1-(4-ethoxyphenyl)-2,2,-difluoro-cyclopropyl-1-methyl amine;
3'-phenoxybenzyl-1-(4-ethoxyphenyl)-2,2-dichlorocyclopropyl-1-methyl amine;
3'-phenoxybenzyl-1-(4-ethoxyphenyl)-2,2-difluorocyclopropyl-1-methyl sulphide;
α-cyano-3'-phenoxybenzyl-1-(4-ethoxyphenyl)-2,2-difluorocyclopropyl-1-methyl sulphide;
3-(3-phenoxyphenyl)-1-[1-(4-ethoxyphenyl)-2,2,3,3-tetrafluorocyclobutyl]propan-1-one; and
α-cyano-3'-phenoxybenzyl-1-(4-ethoxyphenyl)-2,2-difluorocyclopropyl-1-methyl sulphoxide.

8. A compound according to claim 1 which is (−) 3'-phenoxybenzyl-1-(4-ethoxyphenyl)-2,2-difluorocyclopropyl-1-methyl ether,

9. A compound according to claim 1 which is (+) 3'-phenoxybenzyl-1-(4-ethoxyphenyl)-2,2-difluorocyclopropyl-1-methyl ether.

10. An arthropodicidal composition, characterized in that it comprises as an active ingredient a compound according to any preceding claim in admixture with a diluent or carrier.

11. A composition as claimed in claim 10 characterized in that it further includes at least one substance which has a synergistic or intensifying effect on pyrethroids selected from propynyl ethers, propynyl oximes, propynyl carbamates, propynyl phosphonates, S,S,S-tributylphosphorotrithionates, [2-(2-butoxyethoxy)ethoxy]4,5-methylenedioxy-2-propyltoluene (Piperonyl butoxide), 3-hexyl-5-(3,4-methylenedioxyphenyl)-2-cyclohexanone (Piperonyl cyclonene), 2-(3,4-methylenedioxyphenoxy)-3,6,9-trioxaundecane (Sesoxane or Sesamex), 1,2-(methylenedioxy)-4-[2-(octylsulfinyl)propyl)benzene (Sulfoxide), and dipropyl-5,6,7,8-tetrahydro-7-methylnaphtho-[2,3-d]-3-dioxole-5,6-dicarboxylate (n-Propyl isome).

12. A composition as claimed in claim 11 characterized in that the substance is present in the composition in an amount of from 1/100th to fifty times the weight of the compound formula (I).

13. A method of combating arthropods, characterized in that a compound according to any of claims 1 to 9 alone or in the form of a composition as claimed in any one of claims 10 to 12 is applied to the arthropods, or to a habitat thereof.

14. The use of a compound according to any of claims 1 to 9 in the manufacture of a composition for use in a method of freeing or protecting domesticated animals from parasitical insects or acarids by applying said composition to said animals.

**Claims for the Contracting States: DE FR NL SE**

1. A compound of the general formula (I) or an isomeric form thereof:

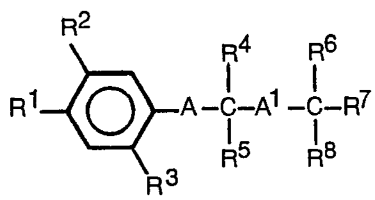

(I)

wherein

$R^1$ is a halo group; or a lower alkyl, lower alkoxy or lower alkylthio group, in each of which the alkyl group may be substituted with one or more halo groups;

$R^2$ is hydrogen or a halo or halomethyl group; or $R^1$ and $R^2$ together form a methylenedioxy, or a difluoro-methylenedioxy group or $R^1$ and $R^2$ together with the carbon atoms to which they are attached form an aromatic ring;

$R^3$ is hydrogen or a halo group;

A is one of the groups X or Y

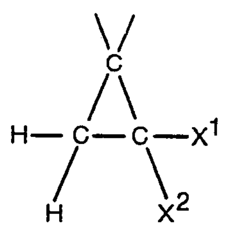

(X)

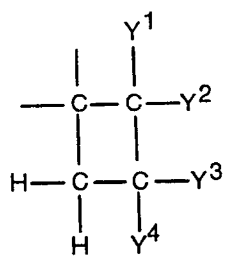

(Y)

wherein $X^1$ and $X^2$ are the same or different and each is a fluoro, chloro, bromo or methyl group, and $Y^1$, $Y^2$, $Y^3$ and $Y^4$ are the same or different and each is hydrogen or a fluoro, bromo, chloro, or methyl group;

$A^1$ is $CH_2$, $CF_2$, $CCl_2$, O, S, SO, NH or NZ where Z is a halo group or

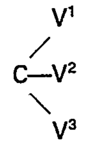

where $V^1$, $V^2$, $V^3$ are the same or different and each is fluorine, chlorine or hydrogen;

$R^4$ is hydrogen, chlorine or fluorine; and

$R^5$ is hydrogen, chlorine or fluorine; or

$R^4$ and $R^5$ together form =O when $A^1$ is $CH_2$; and

$R^6$ is hydrogen, deuterium, CN, or C≡CH; and

$R^7$ is 3-phenoxyphenyl, 2-phenoxy-6-pyridyl, 2-phenoxy-3-fluoro-6-pyridyl, pentafluorophenyl, 4-fluoro-3-phenoxyphenyl, N-pyrollyl-3-benzyl, 3,4-methylene-dioxy phenyl; or 3-(4-methoxyphenoxy)-phenyl;

$R^8$ is hydrogen or deuterium

subject to the proviso that the compound is not 1-(3-phenoxyphenyl)-3-[1-(4-chlorophenyl)-2,2-dichlorocylopropyl)-propane racemate.

2. A compound as claimed in claim 1 subject to the proviso that when $A^1$ is $CH_2$, $R^4$ and $R^5$ cannot both be hydrogen.

3. A compound as claimed in claim 1 characterized in that group $A^1$ is —O—.

4. A compound as claimed in claim 1 characterized in that the group $A^1$ is —S—.

5. A compound as claimed in claim 1 characterized in that the group $A^1$ is —SO—.

6. A compound as claimed in claim 1 characterized in that the group $A^1$ is —NH— or —NZ—.

7. A compound as claimed in claim 1 characterized in that the group $A^1$ is —$CH_2$— and $R^4$ and $R^5$ together form =O.

8. Compound according to claim 1 selected from:

3′-phenoxybenzyl-1-(4-ethoxyphenyl)-2,2,3,3-tetrafluorocyclobutyl-1-methyl ether;
3′-phenoxybenzyl-1-(4-ethoxyphenyl)-2,2-dichlorocyclopropyl-1-methyl ether;
4′-fluoro-3′-phenoxybenzyl-1-(4-ethoxyphenyl)-2,2-difluoro-cyclopropyl-1-methyl ether;
3′-phenoxybenzyl-1-(4-ethoxyphenyl)-2-fluoro-2-chlorocyclopropyl-1-methyl ether;
3′-phenoxybenzyl-1-(4-chlorophenyl)-2,2-difluorocyclopropyl-1-methyl ester;
3′-phenoxybenzyl-1-(3,4-dichlorophenyl)-2,2,3,3-tetrafluorocyclobutyl-1-methyl ether;
pentafluorophenylmethyl-1-(4-ethoxyphenyl)-2,2-dichlorocyclopropyl-1-methyl ether;
3′-phenoxybenzyl-1-(4-ethoxyphenyl)-2,2-difluorocyclopropyl-1-methyl amine;
N-methyl-3′-phenoxybenzyl-1-(4-ethoxyphenyl)-2,2-difluorocyclopropyl-1-methyl amine;
α-cyano-3′-phenoxybenzyl-1-(4-ethoxyphenyl)-2,2,-difluoro-cyclopropyl-1-methyl amine;
3′-phenoxybenzyl-1-(4-ethoxyphenyl)-2,2-dichlorocyclopropyl-1-methyl amine;
3′-phenoxybenzyl-1-(4-ethoxyphenyl)-2,2-difluorocyclopropyl-1-methyl sulphide;
α-cyano-3′-phenoxybenzyl-1-(4-ethoxyphenyl)-2,2-difluorocyclopropyl-1-methyl sulphide;
3-(3-phenoxyphenyl)-1-[1-(4-ethoxyphenyl)-2,2,3,3-tetrafluorocyclobutyl]propan-1-one; and
α-cyano-3′-phenoxybenzyl-1-(4-ethoxyphenyl)-2,2-difluorocyclopropyl-1-methyl sulphoxide.

9. A compound according to claim 1 which is (−) 3′-phenoxybenzyl-1-(4-ethoxyphenyl)-2,2-difluorocyclopropyl-1-methyl ether,

10. A compound according to claim 1 which is (+) 3′-phenoxybenzyl-1-(4-ethoxyphenyl)-2,2-difluorocyclopropyl-1-methyl ether.

11. An arthropodicidal composition, characterized in that it comprises as an active ingredient a compound according to any preceding claim in admixture with a diluent or carrier.

12. A composition as claimed in claim 11 characterized in that it further includes at least one substance which has a synergistic or intensifying effect on pyrethroids selected from propynyl ethers, propynyl oximes, propynyl carbamates, propynyl phosphonates, S,S,S-tributylphosphorotrithionates, [2-(2-butoxy-ethoxy)ethoxy]4,5-methylenedioxy-2-propyltoluene (Piperonyl butoxide), 3-hexyl-5-(3,4-methylenedioxyphenyl)-2-cyclohexanone (Piperonyl cyclonene), 2-(3,4-methylenedioxyphenoxy)-3,6,9-trioxaundecane (Sesoxane or Sesamex), 1,2-(methylenedioxy)-4-[2-(octylsulfinyl)propyl)benzene (Sulfoxide), and dipropyl-5,6,7,8-tetrahydro-7-methylnaphtho-[2,3-d]-3-dioxole-5,6-dicarboxylate (n-Propyl isome).

13. A composition as claimed in claim 12 characterized in that the substance is present in the composition in an amount of from 1/100th to fifty times the weight of the compound formula (I).

14. A method of combating arthropods, characterized in that a compound according to any of claims 1 to 10 alone or in the form of a composition as claimed in any one of claims 11 to 13 is applied to the arthropods, or to a habitat thereof.

15. The use of a compound according to any of claims 1 to 10 in the manufacture of a composition for use in a method of freeing or protecting domesticated animals from parasitical insects or acarids by applying said composition to said animals.

**Patentansprüche für die Vertragsstaaten: BE CH GB IT**

1. Eine Verbindung der allgemeinen Formel (I) oder eine isomerische Form davon:

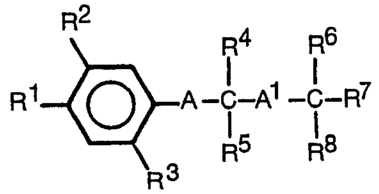

(I)

worin

$R^1$ eine Halogruppe ist; oder eine geringere Alkyl-, geringere Alkoxy- oder geringere Alkylthiogruppe, in welcher jede der Alkylgruppen mit einer oder mehreren Halogruppen ersetzt werden kann;

$R^2$ Wasserstoff oder eine Halo- oder Halomethylgruppe ist; oder $R^1$ und $R^2$ zusammen eine Methylendioxy- oder eine Difluor-methylendioxygruppe bilden oder, $R^1$ und $R^2$ zusammen mit den Kohlenstoffatomen, mit denen sie verbunden sind, einen aromatischen Ring bilden;

$R^3$ Wasserstoff oder eine Halogruppe ist;

A eine der Gruppen X oder Y ist

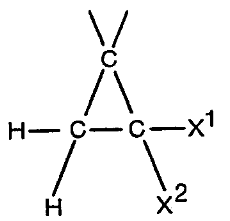

(X)

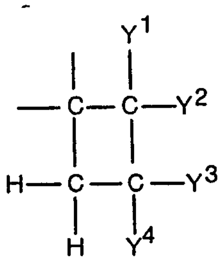

(Y)

worin $X^1$ $X^2$ gleich oder verschieden sind, und jedes eine Fluor-, Brom-, Chlor- oder Methylgruppe ist, und $Y^1$, $Y^2$, $Y^3$ und $Y^4$ gleich oder verschieden sind, und jedes Wasserstoff oder eine Fluor-, Brom-, Chlor- oder Methylgruppe ist;

$A^1$ $CH_2$, $CF_2$, $CCl_2$, O, S, SO, NH oder NZ ist wobei Z eine Halogruppe oder

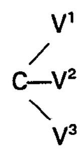

ist, wobei $V^1$, $V^2$, $V^3$ gleich oder verschieden sind und jedes Fluor, Chlor oder Wasserstoff ist;

$R^4$ Wasserstoff, Chlor oder Fluor ist; und

$R^5$ Wasserstoff, Chlor oder Fluor ist; oder

$R^4$ und $R^5$ zusammen =O bilden wenn $A^1$ $CH_2$ ist; und

$R^6$ Wasserstoff, Deuterium, CN oder C≡CH ist; und

$R^7$ 3-Phenoxyphenyl, 2-Phenoxy-6-pyridyl, 2-Phenoxy-3-fluor-6-pyridyl, Pentafluorphenyl, 4-Fluoro-3-phenoxyphenyl, N-Pyrollyl-3-benzyl, 3,4-Methylendioxyphenyl; oder 3-(4-Methoxyphenyl)-phenyl ist;

$R^8$ Waserstoff oder Deuterium ist.

2. Eine Verbindung nach Anspruch 1, dadurch gekennzeichnet, dass Gruppe $A^1$ —O— ist.

3. Eine Verbindung nach Anspruch 1, dadurch gekennzeichnet, dass Gruppe $A^1$ —S— ist.

4. Eine Verbindung nach Anspruch 1, dadurch gekennzeichnet, dass Gruppe $A^1$ —SO— ist.

5. Eine Verbindung nach Anspruch 1, dadurch gekennzeichnet, dass Gruppe $A^1$ —NH— oder —NZ ist.

6. Eine Verbindung nach Anspruch 1, dadurch gekennzeichnet, dass Gruppe $A^1$ —$CH_2$— ist und $R^4$ und $R^5$ zusammen =O bilden.

7. Verbindung nach Anspruch 1, ausgewählt von

3'-Phenoxybenzyl-1-(4-äthoxyphenyl)-2,2,3,3,-tetrafluorcyclobutyl-1-methyläther;

3'-Phenoxybenzyl-1-(4-äthoxyphenyl)-2,2-dichlorocyclopropyl-1-methyläther;

4'-Fluor-3'-phenoxybenzyl-1-(4-äthoxyphenyl)-2,2-difluorcyclopropyl-1-methyläther;

3'-Phenoxybenzyl-1-(4-äthoxyphenyl)-2-fluor-2-chlor-cyclopropyl-1-methyläther;

3'-Phenoxybenzyl-1-(4-chlorphenyl)-2,2-difluor-cyclopropyl-1-methyläther;

3'-Phenoxybenzyl-1-(3,4-dichlorphenyl)-2,2,3,3-tetrafluorcyclobutyl-1-methyläther;

Pentafluorphenylmethyl-1-(4-äthoxyphenyl)-2,2-dichlorcyclopropyl-1-methyläther;

3'-Phenoxybenzoyl-1-(4-äthoxyphenyl)-2,2-difluorcyclopropyl-1-methylamin;

N-Methyl-3'-phenoxybenzyl-1-(4-äthoxyphenyl)-2,2-difluorcyclopropyl-1-methylamin;
α-Cyan-3'-phenoxybenzyl-1-(4-äthoxyphenyl)-2,2-difluorcyclopropyl-1-methylamin;
3'-Phenoxybenzyl-1-(4-äthoxyphenyl)-2,2-dichlorcycyloprppyl-1-methylamin;
3'-Phenoxybenzyl-1-(4-äthoxyphenyl)-2,2-difluorcyclopropyl-1-methylsulphid;
α-Cyan-3'-phenoxybenzyl-1-(4-äthoxyphenyl)-2,2-difluorcyclopropyl-1-methylsulphid;
3-(3-Phenoxyphenyl)-1-[1-(4-äthoxyphenyl)-2,2,3,3-tetrafluorcyclobutyl]propan-1-on; und
α-Cyan-3'-phenoxybenzyl-1-(4-äthoxyphenyl)-2,2,-difluorcyclopropyl-1-methylsulphoxid.

8. Eine Verbindung nach Anspruch 1, welche (−) 3'-Phenoxybenzyl-1-(4-äthoxyphenyl)-2,2-difluorcyclopropyl-1-methyläther ist.

9. Eine Verbindung nach Anspruch 1, welche (+) 3'-Phenoxybenzyl-1-(4-äthoxyphenyl)-2,2-difluorcyclopropyl-1-methyläther ist.

10. Eine arthropozide Zusammensetzung, dadurch gekennzeichnet, dass sie als aktiven Bestandteil eine Verbindung nach einem der vorhergehenden Ansprüche enthält, die einem Vedünnungsmittel oder Träger beigemengt ist.

11. Eine Zusammensetzung nach Anspruch 10, dadurch gekennzeichnet, dass sie weiterhin wenigstens eine Substanz enthält, die einen synergistischen oder verstärkenden Einfluss auf Pyrethroide hat, welche von Propynyläthern, Propynyloximen, Propynylcarbamaten, Propynylphosphonaten, S,S,S-Tributylphosphortrithionaten, [2-(2-Butoxyäthoxy)äthoxy]4,5-methylendioxy-2-propyltoluen (Piperonylbutoxid), 3-Hexyl-5-(3,4-methylendioxphenyl)-2-cyclohexanon (Piperonylcyclonen), 2-(3,4-Methylendioxyphenoxy)-3,6,9-trioxaundecan (Sesoxan oder Sesamex), 1,2-(Methylendioxy)-4-[2-(octylsulfinyl)propyl]benzol (Sulfoxid), und Dipropyl-5,6,7,8-tetrahydro-7-methylnaphto-[2,3-d]-3-dioxol-5,6-dicarboxylat (n-Propylisom) ausgewählt werden.

12. Eine Verbindung nach Anspruch 11, dadurch gekennzeichnet, dass die Substanz in der Zusammensetzung in einem Betrag zwischen 1/100stel und fünfzigmal des Gewichtes der Verbindungsformel (I) enthalten ist.

13. Eine Methode zur Bekämpfung von Arthropoden, dadurch gekennzeichnet, dass eine Verbindung nach einem der Ansprüche 1 bis 9 allein oder in der Form einer Zusammensetzung nach einem der Ansprüche 10 bis 12 auf die Arthropoden oder auf einem derer Sitze angewandt wird.

14. Die Nutzung einer Verbindung nach einem der Ansprüche 1 bis 9 in der Herstellung einer Zusammensetzung zur Nutzung in einer Methode zur Befreiung oder Schützung von Haustieren vor parasitischen Insekten oder Milben, indem die Zusammensetzung auf die Tiere angewandt wird.

**Patentansprüche für die Vertragsstaaten: DE FR NL SE**

1. Eine Verbindung der allgemeinen Formel (I) oder eine isomerische Form davon:

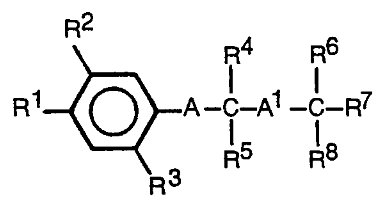

(I)

worin

$R^1$ eine Halogruppe ist; oder eine geringere Alkyl-, geringere Alkoxy- oder geringere Alkylthiogruppe, in welcher jede der Alkylgruppen mit einer oder mehreren Halogruppen ersetzt werden kann;

$R^2$ Wasserstoff oder eine Halo- oder Halomethylgruppe ist; oder $R^1$ und $R^2$ zusammen eine Methylendioxy- oder eine Difluor-methylendioxygruppe bilden oder, $R^1$ und $R^2$ zusammen mit den Kohlenstoffatomen, mit denen sie verbunden sind, einen aromatischen Ring bilden;

$R^3$ Wasserstoff oder eine Halogruppe ist;

A eine der Gruppen X oder Y ist

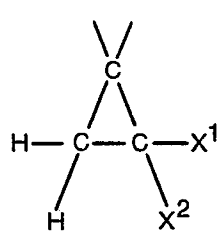

(X)

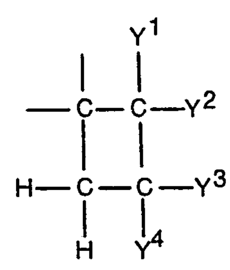

(Y)

worin $X^1$ $X^2$ gleich oder verschieden sind, und jedes eine Fluor-, Brom-, Chlor- oder Methylgruppe ist, und $Y^1$, $Y^2$, $Y^3$ und $Y^4$ gleich oder verschieden sind, und jedes Wasserstoff oder eine Fluor-, Brom-, Chlor- oder Methylgruppe ist;

$A^1$ $CH_2$, $CF_2$, $CCl_2$, O, S, SO, NH oder NZ ist wobei Z eine Halogruppe oder

$$C(V^1)(V^2)(V^3)$$

ist, wobei $V^1$, $V^2$, $V^3$ gleich oder verschieden sind und jedes Fluor, Chlor oder Wasserstoff ist;

$R^4$ Wasserstoff, Chlor oder Fluor ist; und

$R^5$ Wasserstoff, Chlor oder Fluor ist; oder

$R^4$ und $R^5$ zusammen =O bilden wenn $A^1$ $CH_2$ ist; und

$R^6$ Wasserstoff, Deuterium, CN oder C≡CH ist; und

$R^7$ 3-Phenoxyphenyl, 2-Phenoxy-6-pyridyl, 2-Phenoxy-3-fluor-6-pyridyl, Pentafluorphenyl, 4-Fluoro-3-phenoxyphenyl, N-Pyrollyl-3-benzyl, 3,4-Methylendioxyphenyl; oder 3-(4-Methoxyphenyl)-phenyl ist;

$R^8$ Waserstoff oder Deuterium ist, unter der Bedingung, dass die Verbindung nicht 1-(3-Phenoxyphenyl)-3-[1-4-chlorphenyl)-2,2-dichlorocycopropyl]-propanracemat ist.

2. Eine Verbindung nach Anspruch 1, unter der Bedingung dass $R^4$ und $R^5$ nicht beide Wasserstoff sein können wenn $A^1$ $CH_2$ ist.

3. Eine Verbindung nach Anspruch 1, dadurch gekennzeichnet, dass Gruppe $A^1$ —O— ist.

4. Eine Verbindung nach Anspruch 1, dadurch gekennzeichnet, dass Gruppe $A^1$ —S— ist.

5. Eine Verbindung nach Anspruch 1, dadurch gekennzeichnet, dass Gruppe $A^1$ —SO— ist.

6. Eine Verbindung nach Anspruch 1, dadurch gekennzeichnet, dass Gruppe $A^1$ —NH— oder —NZ— ist.

7. Eine Verbindung nach Anspruch 1, dadurch gekennzeichnet, dass Gruppe $A^1$ —$CH_2$— ist und $R^4$ und $R^5$ zusammen =O bilden.

8. Verbindung nach Anspruch 1, ausgewählt von

3′-Phenoxybenzyl-1-(4-äthoxyphenyl)-2,2,3,3-tetrafluorcyclobutyl-1-methyläther.
3′-Phenoxybenzyl-1-(4-äthoxyphenyl)-2,2-dichlorocyclopropyl-1-methyläther;
4′-Fluor-3′-phenoxybenzyl-1-(4-äthoxyphenyl)-2,2-difluorcyclopropyl-1-methyläther;
3′-Phenoxybenzyl-1-(4-äthoxyphenyl)-2-fluor-2-chlor-cyclopropyl-1-methyläther;
3′-Phenoxybenzyl-1-(4-chlorphenyl)-2,2-difluor-cyclopropyl-1-methyläther;
3′-Phenoxybenzyl-1-(3,4-dichlorphenyl)-2,2,3,3-tetrafluorcyclobutyl-1-methyläther;
Pentafluorphenylmethyl-1-(4-äthoxyphenyl)-2,2-dichlorcyclopropyl-1-methyläther;
3′-Phenoxybenzoyl-1-(4-äthoxyphenyl)-2,2-difluorcyclopropyl-1-methylamin;
N-Methyl-3′-phenoxybenzyl-1-(4-äthoxyphenyl)-2,2-difluorcyclopropyl-1-methylamin;
α-Cyan-3′-phenoxybenzyl-1-(4-äthoxyphenyl)-2,2-difluorcyclopropyl-1-methylamin;
3′-Phenoxybenzyl-1-(4-äthoxyphenyl)-2,2-dichlorcycylopropyl-1-methylamin;
3′-Phenoxybenzyl-1-(4-äthoxyphenyl)-2,2-difluorcyclopropyl-1-methylsulphid;
α-Cyan-3′-phenoxybenzyl-1-(4-äthoxyphenyl)-2,2-difluorcyclopropyl-1-methylsulphid;
3-(3-Phenoxyphenyl)-1-[1-(4-äthoxyphenyl)-2,2,3,3-tetrafluorcyclobutyl]propan-1-on; und
α-Cyan-3′-phenoxybenzyl-1-(4-äthoxyphenyl)-2,2,-difluorcyclopropyl-1-methylsulphoxid.

9. Eine Verbindung nach Anspruch 1, welche (−) 3′-Phenoxybenzyl-1-(4-äthoxyphenyl)-2,2-difluorcyclopropyl-1-methyläther ist.

10. Eine Verbindung nach Anspruch 1, welche (+) 3′-Phenoxybenzyl-1-(4-äthoxyphenyl)-2,2-difluorcyclopropyl-1-methyläther ist.

11. Eine arthropozide Zusammensetzung, dadurch gekennzeichnet, dass sie als aktiven Bestandteil eine Verbindung nach einem der vorhergehenden Ansprüche enthält, die einem Vedünnungsmittel oder Träger beigemengt ist.

12. Eine Zusammensetzung nach Anspruch 11, dadurch gekennzeichnet, dass sie weiterhin wenigstens eine Substanz enthält, die einen synergistischen oder verstärkenden Einfluss auf Pyrethroide hat, welche von Propynyläthern, Propynyloximen, Propynylcarbamaten, Propynylphosphonaten, S,S,S-Tributylphosphortrithionaten, [2-(2-Butoxyäthoxy)äthoxy]4,5-methylendioxy-2-propyltoluen (Piperonylbutoxid), 3-Hexyl-5-(3,4-methylendioxphenyl)-2-cyclohexanon (Piperonylcyclonen), 2-(3,4-Methylendioxyphenoxy)-3,6,9-trioxaundecan (Sesoxan oder Sesamex), 1,2-(Methylendioxy)-4-[2-(octylsulfinyl)propyl]benzol (Sulfoxid), und Dipropyl-5,6,7,8-tetrahydro-7-methylnaphto-[2,3-d]-3-dioxol-5,6-dicarboxylat (n-Propylisom) ausgewählt werden.

13. Eine Verbindung nach Anspruch 12, dadurch gekennzeichnet, dass die Substanz in der Zusammensetzung in einem Betrag zwischen 1/100stel und fünfzigmal des Gewichtes der Verbindungsformel (I) enthalten ist.

14. Eine Methode zur Bekämpfung von Arthropoden, dadurch gekennzeichnet, dass eine Verbindung nach einem der Ansprüche 1 bis 10 allein oder in der Form einer Zusammensetzung nach einem der Ansprüche 11 bis 13 auf die Arthropoden oder auf einem derer Sitze angewandt wird.

15. Die Nutzung einer Verbindung nach einem der Ansprüche 1 bis 10 in der Herstellung einer Zusammensetzung zur Nutzung in einer Methode zur Befreiung oder Schützung von Haustieren vor parasitischen Insekten oder Milben, indem die Zusammensetzung auf die Tiere angewandt wird.

**Revendications pour les Etats contractants: BE CH GB IT**

1. Composé selon la formule générale (I) ou sa forme isomérique:

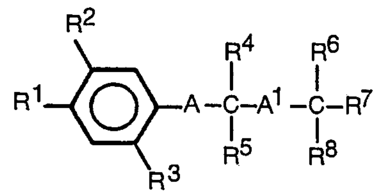

(I)

dont

$R^1$ est un groupe halogène; ou un groupe alcoyle inférieur, alcoxyle inférieur ou thio-alcoyle inférieur, dont le groupe alcoyle est remplacé au besoin par un ou plusieurs groupes halogènes;

$R^2$ est un hydrogène ou groupe halo un halo méthyl; ou $R^1$ et $R^2$ ensemble forment un groupe dioxyméthylène ou difluoro-dioxyméthylène, ou $R^1$ et $R^2$ ensemble avec les atomes de carbone auxquels ils sont reliés forment un anneau aromatique;

$R^3$ est un hydrogène ou groupe halogène;

A est un des groupes X ou Y

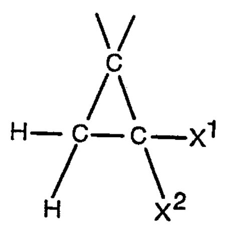

(X)

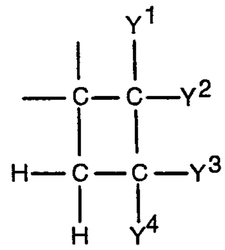

(Y)

dont

$X^1$ et $X^2$ sont indentiques ou différents, chacun étant un groupe fluoro, chloro, bromo ou méthyle, et $Y^1$, $Y^2$, $Y^3$ et $Y^4$ sont indentiques ou différents, chacun étant un hydrogène ou groupe fluoro, chloro, bromo ou méthyle;

$A^1$ est un $CH_2$, $CF_2$ $CCl_2$, O, S, SO, NH ou NZ dont Z est un groupe halo ou

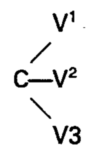

dont $V^1$, $V^2$, $V^3$ sont identiques ou différents, chacun étant un fluor, chlore ou hydrogène;

$R^4$ est un hydrogène, chlore ou fluor; et

$R^5$ est un hydrogène, chlore ou fluor; ou

$R^4$ et $R^5$ ensemble forment =O lorsque $A^1$ est un $CH_2$; et

$R^6$ est un hydrogène, deutérium, CN ou C≡CH; et

$R^7$ est un 3-phénoxyphényle, 2-phénoxy-6-pyridyle, 2-phénoxy-3-fluoro-6-pyridyle, pentafluoro-phényle, 4-fluoro-3-phénoxyphényle, N-pyrrolyl-3-benzyle, 3,4-méthylène-dioxyphényle ou 3-(4-méthoxy-phénoxy)-phényle;

$R^8$ est un hydrogène ou deutérium.

2. Composé selon la lère revendication caractérisé en ce que le groupe $A^1$ est un —O—.

3. Composé selon la lère revendication caractérisé en ce que le groupe $A^1$ est un —S—.

4. Composé selon la lère revendication caractérisé en ce que le groupe $A^1$ est un —SO—.

5. Composé selon la lère revendication caractérisé en ce que le groupe $A^1$ est un —NH— ou —NZ—.

6. Composé selon la lère revendication caractérisé en ce que le groupe $A^1$ est un —$CH_2$— et $R^4$ et $R^5$ ensemble forment un =O.

7. Composé selon la lère revendication sélectionné à partir des composés suivants:

éther 1-(4-éthoxyphényl)-2,2,3,3-tétrafluorocyclobutyl-1-méthylique de 3-phénoxybenzyle;

éther 1-(4-éthoxyphényl)-2,2-dichlorocyclopropyl-1-méthylique de 3-phénoxybenzyle;

éther 1-(4-éthoxyphényl)-2,2,-difluorocyclopropyl-1-méthylique de 4-fluoro-3'-phénoxybenzyle;

éther 1-(4-éthoxyphényl)-2-fluoro-2-chlorocyclopropyl-1-méthylique de 3'-phénoxybenzyle;

éther 1-(4-chlorophényl)-2,2,-difluorocyclopropyl-1-méthylique de 3'-phénoxybenzyle;

éther 1-(3,4-dichlorophényl)-2,2,3,3-tétrafluorocyclobutyl-1-méthylique de 3'-phénoxybenzyle;

éther 1-(4-éthoxyphényl)-2,2-dichlorocyclopropyl-1-méthylique de pentafluorophénylméthyle;

amine 1-(4-éthoxyphényl)-2,2-difluorocyclopropyl-1-méthylique de 3′-phénoxybenzyle;
amine 1-(4-éthoxyphényl)-2,2-difluorocyclopropyl-1-méthylique de N-méthyle-3′-phénoxybenzyle;
amine 1-(4-éthoxyphényl)-2,2-difluorocyclopropyl-1-méthylique de α-cyano-3′-phénoxybenzyle;
amine 1-(4-éthoxyphényl)-2,2-dichlorocyclopropyl-1-méthylique de 3′-phénoxybenzyle;
sulfure 1-(4-éthoxyphényl)-2,2-difluorocyclopropyl-1-méthylique de 3′-phénoxybenzyle;
sulfure 1-(4-éthoxyphényl)-2,2-difluorocyclopropyl-1-méthylique de α-cyano-3′-phénoxybenzyle;
3-(3-phénoxyphényl)-1-{1-(4-éthoxyphényl)-2,2,3,3-tétrafluorocyclobutyle}-propane-1-un; et
sulfoxide 1-(4-éthoxyphényl)-2,2,-difluorocyclopropyl-1-méthylique de α-cyano-3′-phénoxybenzyle.

8. Composé selon la lère revendication qui est éther (−) 1-(4-éthoxyphényl)-2,2-difluorocyclopropyl-1-méthylique de 3′-phénoxybenzyle.

9. Composé selon la lère revendication qui est éther (+) 1-(4-éthoxyphényl)-2,2-difluorocyclopropyl-1-méthylique de 3′-phénoxybenzyle.

10. Composé arthropodicide caractérisé en ce qu'il prévoit comme élément actif un composé selon une des revendications ci-avant ajouté à un produit solvant ou porteur.

11. Composé selon la 10ème revendication caractérisé en ce qui'il prévoit également au moins un élément à effet synergistique ou intensificateur sur les pyréthoïdes sélectionnés à partir d'éthers-, d'oximes, de carbamates, de phosphonates propynyliques, S,S,S-tributylphosphorotriththionates, {2-(2-butoxyéthoxy)éthoxy}4,5-méthylène, dioxy-2-propyltoluène (Pyperonyle butoxide), 3-hexyle-5-(3,4-méthylène dioxiphényle)-2-cyclohexanone (Pyperonyle cyclonène), 2-(3,4-méthylène dioxyphénoxy)-3,6,9-trioxaundecane (Sesoxanone ou Sesamex), 1,2-(méthylène dioxy)-4-{2-(octyle sulfinyl) propyle}benzène (Sulfoxide), et 5,6,7,8-tétrahydro-7-méthyle naphtho-{2,3-d}-3-dioxole-5,6-dicarboxylate de dipropyle (n-propyle isome).

12. Préparation selon la 11ème revendication caractérisée en ce que la substance est présente à raison d'1/100ème à 50 fois le poids de la formule du composé (I).

13. Méthode pour combattre les arthropodes caractérisée en ce qu'un composé sous forme prévue à la lère juqu'à la 9ème la revendication ou en préparation selon l'une ou l'autre de la 10ème à la 12ème revendication est appliquée aux arthropodes ou à leur environnement.

14. Exploitation d'un composé selon l'une ou l'autre de la lère à la 9ème revendication dans l'élaboration d'une préparation pour les animaux domestiques, dont l'application est prévue pour en éliminer les insectes parasites ou acarides ou pour protéger ledits animaux contre lesdits parasites ou acarides.

**Revendications pour les Etats contractants: DE FR NL SE**

1. Composé selon la formule générale (I) ou sa forme isomérique:

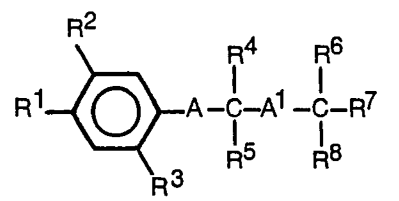

(I)

dont

$R^1$ est un groupe halogène; ou un groupe alcoyle inférieur, alcoxyle inférieur ou thio-alcoyle inférieur, dont le groupe alcoyle est remplacé au besoin par un ou plusieurs groupes halogènes;

$R^2$ est un hydrogène ou groupe halo un halo méthyl; ou $R^1$ et $R^2$ ensemble forment un groupe dioxyméthylène ou difluoro-dioxyméthylène, ou $R^1$ et $R^2$ ensemble avec les atomes de carbone auxquels ils sont reliés forment un anneau aromatique;

$R^3$ est un hydrogène ou groupe halogène;

A est un des groupes X ou Y

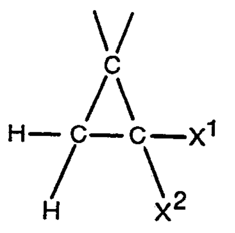

(X)

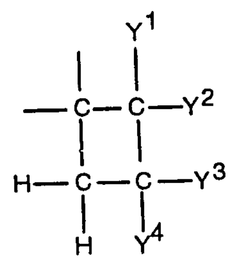

(Y)

dont

$X^1$ et $X^2$ sont indentiques ou différents, chacun étant un groupe fluoro, chloro, bromo ou méthyle, et $Y^1$,

$Y^2$, $Y^3$ et $Y^4$ sont indentiques ou différents, chacun étant un hydrogène ou groupe fluoro, chloro, bromo ou méthyle;

$A^1$ est un $CH_2$, $CF_2$ $CCl_2$, O, S, SO, NH ou NZ dont Z est un groupe halo ou

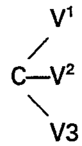

dont $V^1$, $V^2$, $V^3$ sont identiques ou différents, chacun étant un fluor, chlore ou hydrogène;

$R^4$ est un hydrogène, chlore ou fluor; et

$R^5$ est un hydrogène, chlore ou fluor; ou

$R^4$ et $R^5$ ensemble forment =O lorsque $A^1$ est un $CH_2$; et

$R^6$ est un hydrogène, deuterium, CN ou C=CH; et

$R^7$ est un 3-phénoxyphényle, 2-phénoxy-6-pyridyle, 2-phénoxy-3-fluoro-6-pyridyle, pentafluorophényle, 4-fluoro-3-phénoxyphényle, N-pyrrolyl-3-benzyle, 3,4-méthylène-dioxyphényle ou 3-(4-méthoxyphénoxy)-phényle;

$R^8$ est un hydrogène ou deutérium.

sous réserve que le composé ne soit pas un mélange racémique de propane-1-(3-phénoxyphényl)-3-{1-(4-chlorophényl)-2,2-dichlorocyclopropyle}.

2. Composé selon la lère revendication caractérisé en ce que le groupe $A^1$ est un $CH_2$, les deux $R^4$ et $R^5$ ne sont pas des hydrogènes.

3. Composé selon la lère revendication caractérisé en ce que le groupe $A^1$ est un —O—.

4. Composé selon la lère revendication caractérisé en ce que le groupe $A^1$ est un —S—

5. Composé selon la lère revendication caractérisé en ce que le groupe $A^1$ est un —SO—.

6. Composé selon la lère revendication caractérisé en ce que le groupe $A^1$ est un —NH— ou —NZ—.

7. Composé selon la lère revendication caractérisé en ce que le groupe $A^1$ est un —$CH_2$— et $R^4$ et $R^5$ ensemble forment un =O.

8. Composé selon la lère revendication sélectionné à partir des composés suivants:

éther 1-(4-éthoxyphényl)-2,2,3,3-tétrafluorocyclobutyl-1-méthylique de 3-phénoxybenzyle;
éther 1-(4-éthoxyphényl)-2,2-dichlorocyclopropyl-1-méthylique de 3-phénoxybenzyle;
éther 1-(4-éthoxyphényl)-2,2,-difluorocyclopropyl-1-méthylique de 4'-fluoro-3'-phénoxybenzyle;
éther 1-(4-éthoxyphényl)-2-fluoro-2-chlorocyclopropyl-1-méthylique de 3'-phénoxybenzyle;
éther 1-(4-chlorophényl)-2,2,-difluorocyclopropyl-1-méthylique de 3'-phénoxybenzyle;
éther 1-(3,4-dichlorophényl)-2,2,3,3-tétrafluorocyclobutyl-1-méthylique de 3'-phénoxybenzyle;
éther 1-(4-éthoxyphényl)-2,2-dichlorocyclopropyl-1-méthylique de pentafluorophénylméthyle;
amine 1-(4-éthoxyphényl)-2,2-difluorocyclopropyl-1-méthylique de 3'-phénoxybenzyle;
amine 1-(4-éthoxyphényl)-2,2-difluorocyclopropyl-1-méthylique de N-méthyle-3'-phénoxybenzyle;
amine 1-(4-éthoxyphényl)-2,2-difluorocyclopropyl-1-méthylique de α-cyano-3'-phénoxybenzyle;
amine 1-(4-éthoxyphényl)-2,2-dichlorocyclopropyl-1-méthylique de 3'-phénoxybenzyle;
sulfure 1-(4-éthoxyphényl)-2,2-difluorocyclopropyl-1-méthylique de 3'-phénoxybenzyle;
sulfure 1-(4-éthoxyphényl)-2,2-difluorocyclopropyl-1-méthylique de α-cyano-3'-phénoxybenzyle;
3-(3-phénoxyphényl)-1-{1-(4-éthoxyphényl)-2,2,3,3-tétrafluorocyclobutyle}-propane-1-un; et
sulfoxide 1-(4-éthoxyphényl)-2,2,-difluorocyclopropyl-1-méthylique de α-cyano-3'-phénoxybenzyle.

9. Composé selon la lère revendication qui est éther (−) 1-(4-éthoxyphényl)-2,2-difluorocyclopropyl-1-méthylique de 3'-phénoxybenzyle.

10. Composé selon la lère revendication qui est éther (+) 1-(4-éthoxyphényl)-2,2-difluorocyclopropyl-1-méthylique de 3'-phénoxybenzyle.

11. Composé arthropodicide caractérisé en ce qu'il prévoit comme élément actif un composé selon une des revendications ci-avant ajouté à un produit solvant ou porteur.

12. Composé selon la 11ème revendication caractérisé en ce qui'il prévoit également au moins un élément à effet synergistique ou intensificateur sur les pyréthoïdes sélectionnés à partir d'éthers-, d'oximes, de carbamates, de phosphonates propynyliques, S,S,S-tributylphosphorotrithionates, {2-(2-butoxyéthoxy)éthoxy}4,5-méthylène, dioxy-2-propyltoluène (Pyperonyle butoxide), 3-hexyle-5-(3,4-méthylène dioxiphényle)-2-cyclohexanone (Pyperonyle cyclonène), 2-(3,4-méthylène dioxyphénoxy)-3,6,9-trioxaundecane (Sesoxanone ou Sesamex), 1,2-(méthylène dioxy)-4-{2-(octyle sulfinyl) propyle}benzène (Sulfoxide), et 5,6,7,8-tétrahydro-7-méthyle naphtho-{2,3-d}-3-dioxole-5,6-dicarboxylate de dipropyle (n-propyle isome).

13. Préparation selon la 12ème revendication caractérisée en ce que la substance est présente à raison d'1/100ème à 50 fois le poids de la formule du composé (I).

14. Méthode pour combattre les arthropodes caractérisée en ce qu'un composé sous forme prévue à la

1ère juqu'à la 10ème revendication ou en préparation selon l'une ou l'autre de la 11ème à la 13ème revendication est appliquée aux arthropodes ou à leur environnement.

15. Exploitation d'un composé selon l'une ou l'autre de la 1ère à la 10ème revendication dans l'élaboration d'une préparation pour les animaux domestiques, dont l'application est prévue pour en éliminer les insectes parasites ou acarides ou pour protéger ledits animaux contre lesdits parasites ou acarides.